(19) Europäisches Patentamt
European Patent Office
Office européen des brevets

(11) EP 3 685 816 B1

(12) EUROPEAN PATENT SPECIFICATION

(45) Date of publication and mention of the grant of the patent:
**29.05.2024 Bulletin 2024/22**

(21) Application number: **18859902.1**

(22) Date of filing: **20.09.2018**

(51) International Patent Classification (IPC):
***A61K 8/02*** *(2006.01)* ***A61K 8/34*** *(2006.01)*
***A61K 8/41*** *(2006.01)* ***A61Q 5/08*** *(2006.01)*
***A61Q 5/10*** *(2006.01)* ***A61K 8/25*** *(2006.01)*
***A61K 8/22*** *(2006.01)*

(52) Cooperative Patent Classification (CPC):
**A61K 8/022; A61K 8/22; A61K 8/25; A61K 8/345; A61K 8/347; A61K 8/41; A61K 8/416; A61Q 5/08; A61Q 5/10;** A61K 2800/4324

(86) International application number:
**PCT/JP2018/034741**

(87) International publication number:
**WO 2019/059252 (28.03.2019 Gazette 2019/13)**

(54) **POWDERY COMPOSITION WITH REDUCED AMMONIA OCCURRENCE AND AMMONIA MALODOR**

PULVERFÖRMIGE ZUSAMMENSETZUNG MIT REDUZIERTEM AMMONIAKVORKOMMEN UND SCHLECHTEM AMMONIAKGERUCH

COMPOSITION PULVÉRULENTE AVEC UNE PRÉSENCE RÉDUITE D’AMMONIAC ET D’ODEUR D’AMMONIAC

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **20.09.2017 JP 2017180034**
**09.08.2018 JP 2018150485**

(43) Date of publication of application:
**29.07.2020 Bulletin 2020/31**

(73) Proprietor: **Kao Corporation**
**Chuo-Ku**
**Tokyo 103-8210 (JP)**

(72) Inventors:
- **HARUKI, Ryoichi**
**Wakayama-shi**
**Wakayama 640-8580 (JP)**
- **WARITA, Hiroaki**
**Wakayama-shi**
**Wakayama 640-8580 (JP)**
- **FUKUHARA, Masaki**
**Tokyo 131-8501 (JP)**
- **TAKEDA, Kosuke**
**Wakayama-shi**
**Wakayama 640-8580 (JP)**

(74) Representative: **Hoffmann Eitle**
**Patent- und Rechtsanwälte PartmbB**
**Arabellastraße 30**
**81925 München (DE)**

(56) References cited:
**WO-A1-2016/098786**
**DE-A1- 19 824 685**
**FR-A1- 3 015 232**
**JP-A- S5 148 442**
**JP-A- H10 114 635**
**JP-A- 2000 502 713**
**JP-A- 2010 189 336**
**JP-A- 2012 503 675**
**JP-A- 2015 189 709**
**JP-A- 2016 011 297**

- **Madrex: "Ex: beaute Vision Foundation Fine Cover Powder (Pink Ocher 01)", Cosmetic-Info , 1 July 2015 (2015-07-01), pages 1-5, XP009519495, JAPAN Retrieved from the Internet: URL:https://www.cosmetic-info.jp/prod/detail.php?id=38655 [retrieved on 2020-03-23]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

### Field of the Invention

The present invention relates to a powdery composition and a hair dyeing or bleaching kit using the same.

### Background of the Invention

Alkanolamines such as 2-amino-2-methyl-1-propanol are widely used in the cosmetic and pharmaceutical fields as a pH adjusting agent, an emulsifying agent, a neutralizing agent and the like. Among these, 2-amino-2-methyl-1-propanol is used as an alkaline agent in the hair dye field (see, e.g., PTLs 1 and 2).

Among these, PTL 2 discloses that a specific azo dye and 2-amino-2-methyl-1-propanol are used in combination at a specific ratio as a hair dye composition exhibiting high hair dyability of the direct dye therein, suppressed color transfer to fabrics and the like, and moreover, improved solubility of the azo dye in the hair dye composition.

Ammonium halides such as ammonium chloride are used as an alkaline agent in hair dye compositions and the like.

FR 3 015 232 A1 describes a packaging article comprising i) an envelope defining at least one cavity, the envelope comprising water-soluble or liposoluble fibres, ii) at least one anhydrous dye composition containing at least one oxidation dye; and iii) optionally at least one anhydrous oxidizing agent, preferably in powder form; it being understood that the dye composition ii) and the chemical oxidizing agent iii), when it is present, are in one of the cavities of the envelope i).

(PTL 1) JP 2007-320923 A
(PTL 2) JP 2016-11297 A

### Summary of the Invention

The present invention provides a powdery composition comprising components (A), (B), (C) and (D) below, wherein a molar ratio of component (B) to component (A), (B)/(A), is 0.1 or more and 10 or less and the water content is 10 mass% or less:

Component (A): an alkanolamine having an SP value by the Fedors method of 10 $(cal/cm^3)^{1/2}$ or more and 16 $(cal/cm^3)^{1/2}$ or less or a salt thereof;
Component (B): a compound having an SP value by the Fedors method within $\pm 6$ $(cal/cm^3)^{1/2}$ inclusive of the SP value of the component (A) as an alkanolamine (with a proviso that compounds corresponding to component (A) are excluded from the component (B));
Component (C): a powdery carrier;
Component (D): an ammonium halide.

The present invention further provides a hair dyeing or bleaching kit composed of a first composition consisting of the powdery composition above and a second composition comprising hydrogen peroxide.

The present invention further provides use of a mixture of the powdery composition above and hydrogen peroxide for hair.

The present invention further provides a method for manufacturing the powdery composition above, comprising mixing the components (A) to (C) such that the components (A) and (B) are supported on the component (C), and mixing the component (D) therewith.

The present invention further provides a method of dyeing or bleaching hair comprising the following steps (a) to (c):

(a) mixing a first composition consisting of the powdery composition above and a second composition comprising hydrogen peroxide to prepare a mixture;
(b) applying the mixture to hair and leaving the hair to stand for 1 to 45 minutes;
(c) washing the hair to rinse off the mixture.

### Detailed Description of the Invention

The present inventors have investigated to use alkanolamine as a powdery composition supported on a powdery

carrier to mix the powdery composition with an aqueous solution of hydrogen peroxide in order to suppress reactions during storage of the hair dyeing or bleaching composition to enhance dyeing and bleaching ability. However, it has been found that from the powdery composition alkanolamine can volatilize during storage to react with other ingredients such as an ammonium halide, resulting in ammonia occurrence. This volatilization of the alkanolamine causes not only malodor derived from ammonia but also deterioration of dyeability as a hair dyeing agent and bleaching ability as a hair bleaching agent.

The present inventors found that by employing a compound having solubility parameters close to that of alkanolamine on a powdery carrier in combination with alkanolamine, volatilization of alkanolamine due to storage is suppressed, and as a result, it is possible to suppress ammonia occurrence due to reactions between alkanolamine and other compounded ingredients such as an ammonium halide and to suppress deterioration of dyeing/bleaching ability.

The present invention relates to a powdery composition with suppressed volatilization of alkanolamines during storage and also maintained dyeing/bleaching ability when used in combination with an aqueous solution of hydrogen peroxide, and a hair dyeing kit using the same.

[Component (A): specific alkanolamine or a salt thereof]

Component (A) is an alkanolamine having an SP value of 10 $(cal/cm^3)^{1/2}$ or more and 16 $(cal/cm^3)^{1/2}$ or less by the Fedors method, or a salt of the alkanolamine. The SP value of the component (A) refers to the SP value for the alkanolamine. That is, even in the case that the component (A) is a salt of an alkanolamine, the SP value as the free amine form is employed as the SP value of the component (A). The component (A) improves the dyeability of the direct dye in the case that the powdery composition of the present invention is used for hair dyeing.

The Fedors method represents a method of estimating solubility parameter (SP value) from the molecular structure, proposed by Fedors, and the SP value $\delta$ can be given by aggregation energy $E_{coh}$ (cal/mol) and molar molecular volume V $(cm^3/mol)$ for each structural unit according to the following equation.

$$\delta=[\Sigma E_{coh}/\Sigma V]^{1/2}$$

In the equation, $\delta$ represents an SP value, $E_{coh}$ represents aggregation energy (cal/mol), and V represents molar molecular volume $(cm^3/mol)$.

The SP value of the component (A) as an alkanolamine is 10 $(cal/cm^3)^{1/2}$ or more, preferably 11 $(cal/cm^3)^{1/2}$ or more, more preferably 12 $(cal/cm^3)^{1/2}$ or more, and 16 $(cal/cm^3)^{1/2}$ or less, preferably 15 $(cal/cm^3)^{1/2}$ or less, more preferably 14 $(cal/cm^3)^{1/2}$ or less from the viewpoint of solubility in water. The SP value of the component (A) as an alkanolamine is preferably 11 $(cal/cm^3)^{1/2}$ or more and 15 $(cal/cm^3)^{1/2}$ or less, and more preferably 12 $(cal/cm^3)^{1/2}$ or more and 14 $(cal/cm^3)^{1/2}$ or less.

The alkanolamine as the component (A) is preferably a primary alkanolamine. Specific examples of the alkanolamine as the component (A) include, 2-amino-2-methyl-1-propanol (12.22), monoethanolamine (14.26), isopropanolamine (13.05), and 2-aminobutanol (12.40). Among these, 2-amino-2-methyl-1-propanol is preferable from the viewpoint of improving the color quality (numerical values in parentheses indicate the SP value $(cal/cm^3)^{1/2}$ by the Fedors method).

Examples of the alkanolamine salt include inorganic acid salts such as hydrochloride and phosphate; and organic acid salts such as citrate, glycolate and lactate, from the viewpoint of the suitability in formulation.

The alkanolamine or a salt thereof can be used alone or in combination of two or more. In the case that two or more compounds are used in combination as the component (A), the SP value may be calculated according to the following equation.

$$\delta=\delta_1\varphi_1+\delta_2\varphi_2+\cdot\cdot\cdot+\delta_i\varphi_i$$

In the equation, $\delta$ represents an SP value and $\varphi$ represents a volume fraction.

The content of the component (A) in the powdery composition of the present invention is preferably 0.1 mass% or more, more preferably 0.2 mass% or more, even more preferably 0.5 mass% or more, even more preferably 1 mass% or more, and even more preferably 3 mass% or more from the viewpoint of adjusting the pH to a suitable pH range when mixed with the composition containing hydrogen peroxide, and is preferably 15 mass% or less, more preferably 12.5 mass% or less, even more preferably 10 mass% or less, even more preferably 9 mass% by the present invention or less, and even more preferably 8 mass% or less from the viewpoint of storage stability of the formulation. The content of the component (A) is preferably 0.1 mass% or more and 15 mass% or less, more preferably 0.2 mass% or more and 12.5 mass% or less, even more preferably 0.5 mass% or more and 10 mass% or less, even more preferably 1 mass%

or more and 8 mass% or less, and even more preferably 3 mass% or more and 8 mass% or less.

[Component (B): a compound having an SP value different from the SP value of Component (A) in a specific range]

The component (B) has high affinity with the component (A) and can suppress volatilization of the component (A) during storage of the powdery composition. The component (B) is a compound having an SP value by the Fedors method is within ±6 $(cal/cm^3)^{1/2}$ inclusive of the SP value of the component (A) as an alkanolamine (hereinafter, simply referred to as "SP value of the component (A)), but it is preferable that the component (B) have an SP value equal to or higher than the SP value of the component (A) and equal to or lower than the SP value of the component (A) +6 $(cal/cm^3)^{1/2}$. Note that a compound corresponding to the component (A) is excluded from component (B).

The range of the SP value of the component (B) is not self-evident because the range is determined by the relationship with the SP value of the alkanolamine actually used as the component (A). For example, in the case that 2-amino-2-methyl-1-propanol is used as the component (A) (its SP value is 12.22 $(cal/cm^3)^{1/2}$), compounds having an SP value of from 6.22 to 18.22 $(cal/cm^3)^{1/2}$ may be used as the component (B) (with a proviso that an alkanolamine and a salt thereof are excluded from component (B)). The component (B) is preferably in a liquid state at room temperature, preferably has a melting point of 25°C or less, more preferably 20°C or less, and even more preferably 15°C or less. The component (B) is preferably a polyol, more preferably a diol, and more preferably an aliphatic diol or an aromatic diol from the viewpoint of suppressing volatilization of the component (A). The aliphatic diol preferably has 2 or more and 12 or less of carbon atoms, and more preferably has 2 or more and 6 or less of carbon atoms. The aromatic diol preferably has 6 or more and 12 or less of carbon atoms, and more preferably has 6 or more and 9 or less of carbon atoms. Specific examples thereof include ethylene glycol (17.84), diethylene glycol (14.97), propylene glycol (15.91), dipropylene glycol (13.56), 1,3-propanediol (16.14), 1,4-butanediol (14.99), resorcinol (17.38) (numerical values in parentheses indicate SP values calculated by the Fedors method $(cal/cm^3)^{1/2}$).

Compounds corresponding to the component (B) can be used alone or in combination of two or more. In the case that two or more compounds are used in combination as the component (B), the SP value may be calculated according to the equation given in the description for the component (A).

The content of the component (B) in the powdery composition of the present invention is preferably 0.5 mass% or more, more preferably 1 mass% or more, more preferably 3 mass% or more, and even more preferably 5 mass% or more, and preferably 20 mass% or less, more preferably 18 mass% or less, even more preferably 15 mass% or less, and even more preferably 12 mass% or less from the viewpoint of suppressing volatilization of the component (A). The content of the component (B) in the powdery composition of the present invention is preferably 0.5 mass% or more and 20 mass% or less, more preferably 1 mass% or more and 18 mass% or less, even more preferably 3 mass% or more and 15 mass% or less, and even more preferably 5 mass% or more and 12 mass% or less.

The molar ratio of the component (B) to the component (A), (B)/(A), is 0.1 or more and preferably 0.3 or more, from the viewpoint of suppressing volatilization of the component (A) during storage of the powdery composition to suppress ammonia occurrence and the reduction of dyeing/bleaching ability, and is 10 or less, preferably 5 or less, and more preferably 3 or less from the viewpoint of preparing the powdery composition. The molar ratio of the component (B) to the component (A), (B)/(A), is preferably 0.1 or more and 10 or less, more preferably 0.3 or more and 5 or less, and even more preferably 0.3 or more and 3 or less.

[Component (C): powdery carrier]

The powdery carrier as the component (C) is a component which carries the mixture of the components (A) and (B). As the powdery carrier, any of organic and inorganic powders can be used as long as it does not react with the dye, the alkali component, or the components (A) and (B). Specific examples thereof include silica, diatomaceous earth, kaolin, bentonite, starch, tapioca, rice, wheat, potato, nylon powder, montmorillonite, gypsum, sawdust, pearlite, among which diatomaceous earth and silica are preferable. The component (C) can be used alone or in combination of two or more.

The content of the component (C) in the powdery composition of the present invention is preferably 10 mass% or more, more preferably 30 mass% or more, and even more preferably 50 mass% or more from the viewpoint of preparing the composition as a powder, and is preferably 90 mass% or less, more preferably 80 mass% or less, and even more preferably 70 mass% or less from the viewpoint of economic efficiency. The content of the component (C) in the powdery composition of the present invention is preferably 10 mass% or more and 90 mass% or less, more preferably 30 mass% or more and 80 mass% or less, and even more preferably 50 mass% or more and 70 mass% or less.

The mass ratio of the total content of the component (A) and the component (B) to the content of the component (C) in the powdery composition of the present invention, [(A)+(B)]/ (C), is preferably 0.05 or more, more preferably 0.1 or more, even more preferably 0.15 or more, and even more preferably 0.2 or more from the viewpoint of handling when mixed with a composition containing hydrogen peroxide, and preferably 1.5 or less, more preferably 1 or less, even more

preferably 0.5 or less, and even more preferably 0.4 or less from the viewpoint of preparing the composition in a powdery form. The mass ratio of the total content of the component (A) and the total content of the component (B) to the content of the component (C), [(A)+(B)]/(C), is preferably 0.05 or more and 1.5 or less, more preferably 0.1 or more and 1 or less, even more preferably 0.15 or more and 0.5 or less, and even more preferably 0.2 or more and 0.4 or less.

[Component (D): ammonium halide]

The powdery composition of the present invention further contains an ammonium halide as the component (D). The component (D) has a function of promoting the swelling of hair and improving the hair dyeing/bleaching ability. Examples of the ammonium halide include ammonium chloride, ammonium fluoride, ammonium bromide, and ammonium iodide, among which ammonium chloride is preferable. The component (D) can be used alone or in combination of two or more.

The ammonium halide as the component (D) is preferably coated with a hydrophilic polymer compound, from the viewpoint of suppressing reactions with alkanolamine or a salt thereof as the component (A), a solid alkaline agent such as sodium metasilicate which can be separately blended, a direct dye as the component (E), and the like during storage of the powdery composition. The melting point of the hydrophilic polymer compound is preferably 40°C or more, and more preferably 50°C or more, from the viewpoint that the hydrophilic polymer needs to be in a solid state at normal temperature in order to coat the component (D), and is preferably 100°C or less, and more preferably 80°C or less, from the viewpoint of handling. The melting point of the hydrophilic polymer compound is preferably 40°C or more and 100°C or less, and more preferably 50°C or more and 80°C or less. Examples of the hydrophilic polymer compound include a polyoxyalkylene glycol such as polyethylene glycol and polypropylene glycol, and among them, polyethylene glycol is preferable from the viewpoint of storage stability and handling. The number average molecular weight of polyethylene glycol is preferably 1,000 or more, more preferably 3,000 or more, even more preferably 5,000 or more, and preferably 10,000 or less, more preferably 8,000 or less. The number average molecular weight of polyethylene glycol is preferably 1,000 or more and 10,000 or less, more preferably 3,000 or more and 8,000 or less, and even more preferably 5,000 or more and 8,000 or less.

The coating of the component (D) with the hydrophilic polymer compound can be performed by adding the hydrophilic polymer compound heated to the melting point or higher to an ammonium halide, and mixing, and cooling, by using a mixer. Alternatively, warm water having temperature equal to or higher than the melting point of the hydrophilic polymer may be poured through the jacket of the mixer, and a hydrophilic polymer compound having a normal temperature and an ammonium halide may be added thereto and mixed, and cooling may be performed after it is confirmed that the temperature of the mixture has been raised to temperature equal to or higher than the melting point of the hydrophilic polymer.

The coating amount of the hydrophilic polymer compound on the ammonium halide is preferably 1 mass% or more, more preferably 5 mass% or more, and even more preferably 10 mass% or more to the total amount of the ammonium halide used from the viewpoint of storage stability, and preferably 40 mass% or less, more preferably 30 mass% or less, and even more preferably 20 mass% or less from the viewpoint of suppressing generation of coarse grains at the time of coating. The coating amount of the hydrophilic polymer compound on the ammonium halide is preferably 1 mass% or more and 40 mass% or less, more preferably 5 mass% or more and 30 mass% or less, more preferably 10 mass% or more and 20 mass% or less to the total amount of the ammonium halide.

The content of the component (D) in the powdery composition of the present invention is preferably 3 mass% or more, more preferably 5 mass% or more, and even more preferably 10 mass% or more from the viewpoint of hair dyeing/bleaching ability when used for hair dyeing/bleaching, and preferably 30 mass% or less, more preferably 20 mass% or less, and even more preferably 15 mass% or less from the viewpoint of storage stability of the powdery composition. The content of the component (D) in the powdery composition of the present invention is preferably 3 mass% or more and 30 mass% or less, more preferably 5 mass% or more and 20 mass% or less, and even more preferably 10 mass% or more and 15 mass% or less. The content of the component (D) herein does not include the amount of the hydrophilic polymer compound covering the ammonium halide, and refers to the content of the ammonium halide per se.

[Component (E): direct dye]

In the case that the powdery composition of the present invention is used for dyeing hair, the powdery composition may contain a direct dye as the component (E). As the direct dye as the component (E), at least one or more dyes selected from the group consisting of the following azo dyes (E-1), (E-2) and (E-3), acidic dyes, basic dyes, nitro dyes, and disperse dyes can be used.

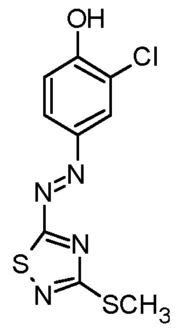

(E-1)

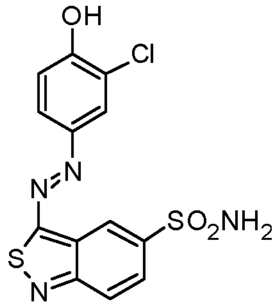

(E-2)

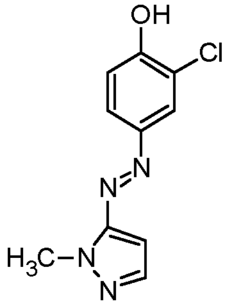

(E-3)

Examples of the acidic dye include Blue No. 1, Violet No. 401, Black No. 401, Orange No. 205, Red No. 227, Red No. 106, Yellow No. 203, and Acid Orange 3.

Examples of the basic dye include Basic Red 51, Basic Blue 99, Basic Brown 16, Basic Brown 17, Basic Red 76, and Basic Yellow 57.

Examples of nitro dyes include 2-nitro-p-phenylenediamine, 2-amino-6-chloro-4-nitrophenol, 3-nitro-p-hydroxyethylaminophenol, 4-nitro-o-phenylenediamine, 4-amino-3-nitrophenol, 4-hydroxypropylamino-3-nitrophenol, N,N-bis(2-hydroxyethyl)-2-nitro-p-phenylenediamine, HC Blue 2, HC Orange 1, HC Red 1, HC Red 3, HC Yellow 2, HC Yellow 4, HC Yellow 5.

Examples of the disperse dyes include Disperse Violet 1, Disperse Blue 1, and Disperse Black 9.

The direct dye as the component (E) can be used alone or in combination of two or more. As the component (E), it is preferable to use at least one azo dye selected from the group consisting of (E-1), (E-2) and (E-3) from the viewpoint of obtaining a bright hair color when applied to hair. The content of the component (E) in the powdery composition of the present invention is preferably 0.001 mass% or more, more preferably 0.005 mass% or more, even more preferably 0.01 mass% or more, even more preferably 0.1 mass% or more, even more preferably 0.5 mass% or more, even more preferably 1.0 mass% or more, and preferably 10 mass% or less, more preferably 9 mass% or less, even more preferably 7.5 mass% or less, even more preferably 5.0 mass% or less, and even more preferably 3.0 mass% or less. The content of the component (E) in the powdery composition of the present invention is preferably 0.001 mass% or more and 10 mass% or less, more preferably 0.005 mass% or more and 9 mass% or less, even more preferably 0.01 mass% or more and 7.5 mass% or less, even more preferably 0.1 mass% or more and 5.0 mass% or less, even more preferably 0.5 mass% or more and 3.0 mass% or less, and even more preferably 1.0 mass% or more and 3.0 mass% or less.

[Component (F): solid alkaline agent]

In the powdery composition of the present invention, it is preferable further to contain a solid alkaline agent as the component (F) in order to improve the bleaching ability by adjusting the pH of the mixture with the oxidizing agent aqueous solution to alkalinity, and further to improve the dyeability in the case that the azo dyes (E-1) to (E-3) described above are contained as the component (E).

Examples of the solid alkaline agent include sodium metasilicate, sodium carbonate, potassium carbonate, ammonium carbonate, guanidine carbonate, sodium bicarbonate, potassium bicarbonate, ammonium bicarbonate and guanidine bicarbonate, among which sodium metasilicate is preferable.

Any of the (F) solid alkaline agents can be used alone or in combination of two or more. The content of the component (F) in the powdery composition of the present invention is preferably 1 mass% or more, more preferably 2 mass% or more, and even more preferably 3 mass% or more from the viewpoint of enhancing the bleaching ability, and is preferably 40 mass% or less, more preferably 30 mass% or less, and even more preferably 25 mass% or less from the viewpoint of economic efficiency. The content of the component (F) in the powdery composition of the present invention is preferably 1 mass% or more and 40 mass% or less, more preferably 2 mass% or more and 30 mass% or less, and even more preferably 3 mass% or more and 25 mass% or less.

[Water]

Although the powdery composition of the present invention may contain water, it is preferable to contain the water content as little as possible from the viewpoint of maintaining the powdery form. The content of water in the powdery composition of the present invention is 0 mass% or more and 10 mass% or less, preferably 0 mass% or more and 7 mass% or less, more preferably 0 mass% or more and 5 mass% or less, more preferably 0 mass% or more and 3 mass%

or less, and even more preferably 0 mass% or more and 1 mass% or less.

[Other optional components]

To the powdery composition of the present invention, other components commonly used as a cosmetic raw material can be further added as long as the powdery form and the function as a hair dyeing/bleaching agent are not deteriorated. The purposes of blending such optional components may include preservation, sequestration, stabilization, prevention from oxidation, ultraviolet absorption, moisturization, coloration on products, flavoring, and specific optional components include an animal and vegetable oil/fat, a higher fatty acid, a protein hydrolysate, a protein derivative, an amino acid, a plant extract, a vitamin, a dye and a perfume.

[Manufacturing Method]

The powdery composition of the present invention can be manufactured, for example, by mixing components (A), (B) and (C) and optional components as appropriate. Any general-purpose mixer can be used for mixing, but a mixer having a high stirring force is preferable from the viewpoint of the mixing property of the components (A), (B) and (C). Preferable examples of the mixer include a Henschel mixer and the like. In the case that the mixture of the components (A) and (B) is in a solid state at ambient temperature, warm water having temperature equal to or above the melting point of the mixture may be poured into the jacket of the mixer. However, from the viewpoint of suppressing volatilization of the component (A), it is preferable to perform the treatment at as low temperature as possible. First, it is preferable to charge the component (C) into the mixer, and then to add a premixed mixture of the component (A) and the component (B), and to perform mixing.

In the case that the powdery composition of the present invention contains the component (E), the manufacture thereof includes a method in which the component (E) is mixed with the component (A) and the component (B) in advance and then the mixture is added into a mixer, and a method in which the component (A) and the component (B) are added into a mixer separately from the component (E). Since the component (E) has low miscibility with the component (A) and the component (B), it is preferable that the component (A) and the component (B) be added into the mixer separately from the component (E) from the viewpoint of mixing uniformly.

It is preferable that the components (A) and (B) be supported on the component (C), and then mixed with the component (D), from the viewpoint of suppressing the reaction between the component (D) and the component (A). In the case that the component (F) is further blended, it is preferable not to add the component (F) simultaneously with the component (D) from the viewpoint of suppressing the reaction with the component (D).

In the case that the powdery composition of the present invention contains (D) an ammonium halide coated with a hydrophilic polymer compound, (E) a direct dye, (F) a solid alkaline agent and the like, the powdery composition of the present invention may be prepared by mixing the components (A), (B), (C), (E) and (F) and other optional components to obtain a first powdery composition, separately by mixing the component (D) with a hydrophilic polymer compound to obtain a second powdery composition consisting of the component (D) coated with the hydrophilic polymer compound, and then by mixing the first and second powdery compositions.

[Usage]

The powdery composition of the present invention can be used by mixing with a composition containing hydrogen peroxide immediately before use and then by applying it to the hair, for dyeing or bleaching hair.

That is, dyeing or bleaching hair by using the powdery composition of the present invention can be carried out by the method having the following steps (a) to (c):

(a) mixing a first composition consisting of the powdery composition of the present invention and a second composition comprising hydrogen peroxide to prepare a mixture;
(b) applying the mixture to hair and leaving the hair to stand for 1 to 45 minutes;
(c) washing the hair to rinse off the mixture.

The composition comprising hydrogen peroxide contains water as a medium, and is preferably acidified. The content of hydrogen peroxide in the composition comprising hydrogen peroxide is preferably 1 mass% or more, more preferably 2 mass% or more, and even more preferably 3 mass% or more, and preferably 20 mass% or less, more preferably 15 mass% or less, and even more preferably 12 mass% or less. The pH of the composition comprising hydrogen peroxide is preferably 5 or less, and more preferably 4 or less, and preferably 1 or more, and more preferably 2 or more.

The composition can contain, in addition to the hydrogen peroxide, other components commonly used as a

cosmetic raw material. Such optional components include a natural or synthetic polymer, a hydrolyzed protein, an amino acid, an antiseptic agent, a chelating agent, a stabilizing agent, an antioxidant, a plant extract, a vitamin, a thickener, a perfume, and an ultraviolet absorber.

[Hair Dyeing or Bleaching Kit]

In view of the above usage embodiments, it is also preferable to provide them in the form of hair dyeing or bleaching kit composed of a first composition consisting of the powdery composition of the present invention and a second composition comprising hydrogen peroxide.

Examples

Reference Experiment

Powdery ammonium chloride (20 g) was put in a sealed bottle (0.5 L, manufactured by Seisho Co., Ltd.) and stored at 50°C for 1 month with the lid closed. The lid of the container after storage was opened, and the presence or absence of ammonia odor was checked, but ammonia odor was not perceived.

Similarly, powdery ammonium chloride (20 g) was put in a 0.5 L sealed bottle (manufactured by Seisho Co., Ltd.) and a PET-screw vial (30 mL, manufactured by Nikko Hansen & Co., Ltd.) containing 2-amino-2-methyl-1-propanol (20 g) was put in the same sealed bottle with the lid of the PET-screw vial opened, and the sealed bottle was stored at 50°C for 1 month without direct contact between ammonium chloride and 2-amino-2-methyl-1-propanol. After the storage, the lid of the container was opened, and the presence or absence of ammonia odor was confirmed, and it was confirmed that ammonia odor was present.

This experiment suggests that a reaction of the vaporized 2-amino-2-methyl-1-propanol with ammonium chloride cause ammonia occurrence.

Examples 1 to 14 and Comparative Examples 1 to 4

Powdery compositions consisting of the compositions shown in Tables 1 and 2 for hair dyeing were prepared by the following method. The obtained powdery composition was evaluated for suppressing volatilization of the component (A) and suppressing fading of the powdery composition by the following method. These results are given in Table 1 altogether.

[Manufacturing method]

Powdery Compositions of Examples 1 to 3 and 5 to 14

Component (C), component (F), and paraffinic oil were put in a Food mixer (MB-MM22G) manufactured by Yamamoto Electric Corporation, and the mixture was agitated for 30 seconds. A mixture of the component (A) and the component (B) was then added therein and the mixture was agitated for 3 minutes. To avoid adverse reactions, the components (D) and (E) were added before each evaluation test to complete the powdery composition.

Powdery Composition of Example 4

A powdery composition was prepared by the method consisting of the following steps (I) and (II).

Step (I)

Component (D) was put in a Henschel mixer (manufactured by Nippon Coke & Engineering Co., Ltd., Mitsui FM Mixer Model: FM5RC/1) in which warm water of 80°C was poured through the jackets, and mixed for 1 minute. Subsequently, polyethylene glycol heated to 80°C was added dropwise. After the addition, the mixture was discharged from the mixer to be put in a high-speed mixer (type: LFS-GS-2J) manufactured by Earthtechnica Co., Ltd., mixed and cooled until the product temperature reached 40°C, and a component (D) coated with polyethylene glycol was obtained.

Step (II)

A component (C), a component (F), and paraffinic oil were put in a MB-MM22G manufactured by Yamamoto Electric Corporation, and these were mixed for 30 seconds. A mixture of a component (A) and a component (B) was

then added and the mixture was agitated for 3 minutes. A component (D) coated with polyethylene glycol obtained in step (I) and a component (E) were added before each evaluation test to obtain a powdery composition, in order to avoid adverse reactions.

Powdery Compositions of Comparative Examples 1 to 3

Powdery compositions were obtained in the same manner as in Examples 1 to 3 and 5 to 14 except that a component (B) was not blended.

Powdery Composition of Comparative Example 4

A powdery composition was obtained in the same manner as in Example 4 except that a component (B) was not blended.

[Evaluation method]

•Suppressing Volatilization of Component (A) (Suppressing Ammonia Occurrence)

The hair dye compositions were put in a vacuum vial SVF-100 manufactured by Nichiden-Rika Glass Co., Ltd. and stored at 50°C for 1 day. The ammonia accumulated in the container after storage was measured by the following procedure. A 3HM manufactured by Gastec Corporation was used as the detection tube. In this evaluation, the concentration of ammonia after storage is directly confirmed, but considering the results of the reference experiment, it can be said that the degree of volatilization of 2-amino-2-methyl-1-propanol by storage is indirectly evaluated.

1. Trim off the end of the sampling bag for gas collection with scissors.
2. Put the container containing the sample from the place where the sample was cut off in 1.
3. Close the location of 1 with a heat seal.
4. Connect the bag outlet to a vacuum pump to completely evacuate the air in the bag.
5. Charge a predetermined amount of air with a syringe.
6. Open the lid of the container for 30 seconds from the outside of the bag, and close again.
7. Connect the bag outlet to the end-folded detector tube.
8. Pull the handle of the gas sampler to perform the measurement.

•Suppressing Discoloration of Powdery composition

The powdery compositions immediately after preparation and the powdery composition after storage in the above-mentioned vacuum vial SVF-100 at 50°C for one month were subjected to colorimetrical measurement using a spectrophotometer CM-700d manufactured by Konica Minolta, Inc. For the measurement, a powder cell, which was an optional accessory, was used. The color change of the powdery composition before and after storage was determined by the following formula. Lower ΔE* value indicates less color fading, which shows higher stability.

$$\Delta E^* = [(\Delta L^*)^2 + (\Delta a^*)^2 + (\Delta b^*)^2]^{1/2}$$

Table 1

| Component (mass%) | | Examples | | | | Comparative Example | | | |
|---|---|---|---|---|---|---|---|---|---|
| | | 1 | 2 | 3 | 4 | 1 | 2 | 3 | 4 |
| (A) | 2-Amino-2-methyl-1-propanol (SP value: 12.22) | 6.7 | 6.7 | 6.7 | 6.5 | 6.7 | 6.7 | 6.7 | 6.5 |
| (B) | Dipropylene glycol (SP value: 13.56) | 10.0 | 10.0 | 10.0 | 9.8 | - | - | - | - |
| (C) | Diatomaceous earth | 47.6 | 47.6 | 47.6 | 46.7 | 57.6 | 57.6 | 57.6 | 56.5 |
| | Fumed silica | 2.2 | 2.2 | 2.2 | 2.2 | 2.2 | 2.2 | 2.2 | 2.2 |

(continued)

| Component (mass%) | | Examples | | | | Comparative Example | | | |
|---|---|---|---|---|---|---|---|---|---|
| | | 1 | 2 | 3 | 4 | 1 | 2 | 3 | 4 |
| (D) | Ammonium chloride | 11.1 | 11.1 | 11.1 | 10.9 | 11.1 | 11.1 | 11.1 | 10.9 |
| (E) | Azo dye (E-1) | - | 1.1 | - | - | - | 1.1 | - | - |
| | Azo dye (E-2) | 1.1 | - | - | 1.1 | 1.1 | - | - | 1.1 |
| | Azo dye (E-3) | - | - | 1.1 | - | - | - | 1.1 | - |
| (F) | Sodium metasilicate | 11.1 | 11.1 | 11.1 | 10.9 | 11.1 | 11.1 | 11.1 | 10.9 |
| | Paraffin oil | 10.2 | 10.2 | 10.2 | 10.0 | 10.2 | 10.2 | 10.2 | 10.0 |
| | Polyethylene Glycol | - | - | - | 1.9 | - | - | - | 1.9 |
| Total | | 100.0 | 100.0 | 100.0 | 100.0 | 100.0 | 100.0 | 100.0 | 100.0 |
| Molar ratio (B)/ (A) (mol/mol) | | 1.0 | 1.0 | 1.0 | 1.0 | - | - | - | - |
| Evaluation | Ammonia concentration (mass%) | 0.8 | 0.9 | 0.8 | 0.9 | 3.5 | 3.5 | 3.5 | 2.9 |
| | Fading ΔE * of powdery compositions | 3.9 | 2.5 | 3.7 | 2.8 | 18.1 | 7.3 | 20.5 | 10.6 |

Table 2

| Component (mass%) | | Examples | | | | | | | | | |
|---|---|---|---|---|---|---|---|---|---|---|---|
| | | 5 | 6 | 7 | 8 (1) | 9 | 10 | 11 | 12 | 13 | 14 |
| (A) | 2-Amino-2-methyl-1-propanol (SP value: 12.22) | 6.7 | 6.7 | 6.7 | 6.7 | 6.7 | 6.7 | 6.7 | 6.7 | 6.7 | 6.7 |
| (B) | Ethylene glycol (SP value: 17.84) | 4.6 | - | - | - | - | - | - | - | - | - |
| | Diethylene glycol (SP value: 14.97) | - | 7.9 | - | - | - | - | - | - | - | - |
| | Propylene glycol (SP value: 15.91) | - | - | 5.7 | - | - | - | - | - | - | - |
| | Dipropylene glycol (SP value: 13.56) | - | - | - | 10.0 | - | - | - | 2.5 | 15.1 | 20.1 |
| | 1,3-propanediol (SP value: 16.14) | - | - | - | - | 5.7 | - | - | - | - | - |
| | 1,4-butanediol (SP value: 14.99) | - | - | - | - | - | 6.7 | - | - | - | - |
| | Resorcinol (SP value: 17.38) | - | - | - | - | - | - | 8.3 | - | - | - |
| (C) | Diatomaceous earth | 52.9 | 49.6 | 51.9 | 47.6 | 51.9 | 50.9 | 49.3 | 55.0 | 42.5 | 37.5 |
| | Fumed silica | 2.2 | 2.2 | 2.2 | 2.2 | 2.2 | 2.2 | 2.2 | 2.2 | 2.2 | 2.2 |
| (D) | Ammonium chloride | 11.1 | 11.1 | 11.1 | 11.1 | 11.1 | 11.1 | 11.1 | 11.1 | 11.1 | 11.1 |
| (E) | Azo dye (E-2) | 1.1 | 1.1 | 1.1 | 1.1 | 1.1 | 1.1 | 1.1 | 1.1 | 1.1 | 1.1 |
| (F) | Sodium metasilicate | 11.1 | 11.1 | 11.1 | 11.1 | 11.1 | 11.1 | 11.1 | 11.1 | 11.1 | 11.1 |
| | Paraffin oil | 10.2 | 10.2 | 10.2 | 10.2 | 10.2 | 10.2 | 10.2 | 10.2 | 10.2 | 10.2 |
| Total | | 100.0 | 100.0 | 100.0 | 100.0 | 100.0 | 100.0 | 100.0 | 100.0 | 100.0 | 100.0 |
| Molar ratio (B)/ (A) (mol/mol) | | 1.0 | 1.0 | 1.0 | 1.0 | 1.0 | 1.0 | 1.0 | 0.25 | \| 1.5 | 2.0 |
| Evaluation | Ammonia concentration (mass%) | 1.4 | 1.7 | 1.1 | 0.8 | 2.0 | 2.3 | 0.2 | 1.1 | \| 0.6 | 0.3 |

Examples 15 to 16 and Comparative Examples 5 to 6

A powdery composition consisting of the compositions shown in Table 3 for bleaching hair was prepared by the following method. The change in hair bleaching ability due to the preservation of the obtained powdery composition was evaluated. The results are shown in Table 3.

[Manufacturing method]

Powdery composition of Example 15

Powdery compositions were obtained in the same manner as in Examples 1 to 3 and 5 to 14 except that Component (E) was not blended.

Powdery composition of Example 16

A powdery composition was obtained in the same manner as in Example 4 except that component (E) was not blended.

Powdery composition of Comparative Example 5

A powdery composition was obtained in the same manner as in Comparative Examples 1 to 3 except that the component (E) was not blended.

Powdery composition of Comparative Example 6

A powdery composition was obtained in the same manner as in Comparative Example 4 except that Component (E) was not blended.

[Evaluation method]

Hair bleaching ability

The hair bleaching ability of the powdery composition immediately after preparation and the powdery composition after being put in the vacuum vial SVF-100 and stored at 50°C for 1 month were compared.

A paste obtained by mixing the powdery composition and the Topchic Developer Lotion-6 % (containing 6 mass% of hydrogen peroxide) manufactured by Goldwell in a mass ratio of 1:2 was applied to a hair tress of a Chinese white hair 1 g in a bath ratio (agent: hair) of 1:1 (mass ratio), the paste was left at 30°C for 30 minutes, and then rinsed with water at about 40°C, washed with a commercial shampoo, and washed with water to rinse off the mixture. A commercial hair rinse was then applied followed by rinsing with water at about 40°C, wiping with a towel, and drying with a dryer.

The color shade of the obtained hair tress immediately after dyeing was measured using a spectrocolorimeter CM-700d manufactured by Konica Minolta, and the difference from the color shade before dyeing was obtained by the above equation to obtain the bleaching ability $\Delta E^*$. The difference ($\Delta E^*_{after\ storage}$ $-DE^*_{before\ storage}$) between the bleaching ability $\Delta E^*_{after\ storage}$ of the powdery composition after storage and the bleaching ability $\Delta E^*_{before\ storage}$ of the powdery composition before storage is closer to 0 indicates that the decrease of the bleaching ability is suppressed.

Table 3

| Component (mass%) | | Examples | | Comparative Example | |
|---|---|---|---|---|---|
| | | 15 | 16 | 5 | 6 |
| (A) | 2-Amino-2-methyl-1-propanol (SP value: 12.22) | 6.7 | 6.6 | 6.7 | 6.6 |
| (B) | Dipropylene glycol (SP value: 13.56) | 10.2 | 9.9 | - | - |
| (C) | Diatomaceous earth | 48.0 | 47.1 | 58.5 | 57.0 |
| | Fumed silica | 2.2 | 2.2 | 2.2 | 2.2 |

(continued)

| Component (mass%) | | Examples | | Comparative Example | |
|---|---|---|---|---|---|
| | | 15 | 16 | 5 | 6 |
| (D) | Ammonium chloride | 11.3 | 11.1 | 11.2 | 11.1 |
| (F) | Sodium metasilicate | 11.3 | 11.1 | 11.2 | 11.1 |
| | Paraffin oil | 10.3 | 10.1 | 10.2 | 10.1 |
| | Polyethylene Glycol | - | 1.9 | - | 1.9 |
| Total | | 100.0 | 100.0 | 100.0 | 100.0 |
| Molar ratio (B)/ (A) (mol/mol) | | 1.0 | 1.0 | - | - |
| Evaluation | Change in hair bleaching ability ($\Delta E^*_{after\ storage} - \Delta E^*_{before\ storage}$) | -2.8 | -0.5 | -4.1 | -4.0 |

**Claims**

1. A powdery composition comprising the following components (A), (B), (C) and (D), wherein a molar ratio of component (B) to component (A), (B) / (A), is 0.1 or more and 10 or less and the water content is 10 mass% or less:

   Component (A): an alkanolamine having an SP value by the Fedors method of 10 $(cal/cm^3)^{1/2}$ or more and 16 $(cal/cm^3)^{1/2}$ or less or a salt thereof;
   Component (B) : a compound having an SP value by the Fedors method within $\pm 6$ $(cal/cm^3)^{1/2}$ inclusive of the SP value of the component (A) as an alkanolamine, with a proviso that compounds corresponding to component (A) are excluded from component (B) ;
   Component (C): a powdery carrier;
   Component (D): an ammonium halide.

2. The powdery composition according to claim 1, wherein the mixture of the component (A) and the component (B) is supported on the component (C).

3. The powdery composition according to claim 1, wherein the component (D) is coated with a hydrophilic polymer compound.

4. The powdery composition according to any one of claims 1 to 3, wherein the component (B) is an aliphatic diol or an aromatic diol.

5. The powdery composition according to any one of claims 1 to 4, which is used for bleaching hair as a mixture with a composition containing hydrogen peroxide.

6. The powdery composition according to any one of claims 1 to 4, further comprising the following component (E): Component (E): a direct dye.

7. The powdery composition according to claim 6, wherein the component (E) is one or more azo dyes selected from the group consisting of (E-1), (E-2) and (E-3) below.

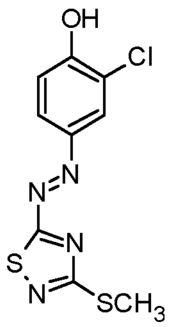
(E-1)

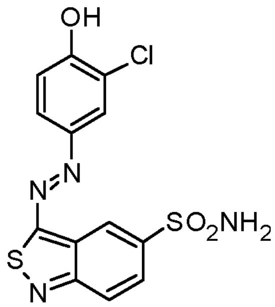
(E-2)

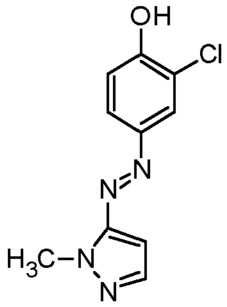
(E-3)

**8.** The powdery composition according to claim 6 or 7, which is used for dyeing hair as a mixture with a composition containing hydrogen peroxide.

**9.** The powdery composition according to any one of claims 1 to 8, further comprising the following component (F): Component (F): a solid alkaline agent.

**10.** The powdery composition according to claim 9, wherein the component (F) is sodium metasilicate.

**11.** A hair dyeing or bleaching kit composed of a first composition consisting of the powdery composition as defined in any one of claims 1 to 10 and a second composition comprising hydrogen peroxide.

**12.** Use of a mixture of the powdery composition according to any one of claims 1 to 10 and a composition comprising hydrogen peroxide for dyeing or bleaching hair.

**13.** A method for manufacturing the powdery composition according to any one of claims 1 to 10, comprising mixing the components (A) to (C) such that the components (A) and (B) are supported on the component (C), and mixing the component (D) therewith.

**14.** A method of dyeing or bleaching hair comprising the following steps (a) to (c):

(a) mixing a first composition consisting of the powdery composition as defined in any one of claims 1 to 10 and a second composition comprising hydrogen peroxide to prepare a mixture;
(b) applying the mixture to hair and leaving the hair to stand for 1 to 45 minutes;
(c) washing the hair to rinse off the mixture.

## Patentansprüche

**1.** Pulverförmige Zusammensetzung, umfassend die folgenden Komponenten (A), (B), (C) und (D), wobei ein Molverhältnis von der Komponente (B) zu der Komponente (A), (B)/(A), 0,1 oder mehr und 10 oder weniger beträgt und der Wassergehalt 10 Massen-% oder weniger beträgt:

Komponente (A): ein Alkanolamin mit einem SP-Wert nach der Fedors-Methode von 10 $(cal/cm^3)^{1/2}$ oder mehr und 16 $(cal/cm^3)^{1/2}$ oder weniger oder ein Salz davon;
Komponente (B): eine Verbindung mit einem SP-Wert nach der Fedors-Methode innerhalb von $\pm 6$ $(cal/cm^3)^{1/2}$ einschließlich des SP-Wertes der Komponente (A) als Alkanolamin, mit der Maßgabe, dass Verbindungen, die der Komponente (A) entsprechen, von der Komponente (B) ausgeschlossen sind;
Komponente (C): ein pulverförmiger Träger;
Komponente (D): ein Ammoniumhalogenid.

**2.** Pulverförmige Zusammensetzung gemäß Anspruch 1, wobei das Gemisch von der Komponente (A) und der Komponente (B) auf der Komponente (C) geträgert ist.

**3.** Pulverförmige Zusammensetzung gemäß Anspruch 1, wobei die Komponente (D) mit einer hydrophilen Polymerverbindung beschichtet ist.

4. Pulverförmige Zusammensetzung gemäß einem der Ansprüche 1 bis 3, wobei die Komponente (B) ein aliphatisches Diol oder ein aromatisches Diol ist.

5. Pulverförmige Zusammensetzung gemäß einem der Ansprüche 1 bis 4, die als Mischung mit einer Wasserstoffperoxid enthaltenden Zusammensetzung zum Bleichen von Haaren verwendet wird.

6. Pulverförmige Zusammensetzung gemäß einem der Ansprüche 1 bis 4, ferner umfassend die folgende Komponente (E):
Komponente (E): einen Direktfarbstoff.

7. Pulverförmige Zusammensetzung gemäß Anspruch 6, wobei die Komponente (E) ein oder mehrere Azofarbstoffe, ausgewählt aus der Gruppe, bestehend aus untenstehenden (E-1), (E-2) und (E-3), ist.

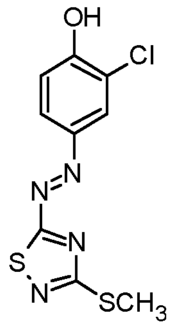
(E-1)

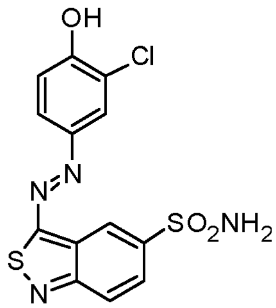
(E-2)

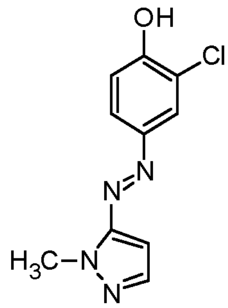
(E-3)

8. Pulverförmige Zusammensetzung gemäß Anspruch 6 oder 7, die als Mischung mit einer Wasserstoffperoxid enthaltenden Zusammensetzung zum Färben von Haaren verwendet wird.

9. Pulverförmige Zusammensetzung gemäß einem der Ansprüche 1 bis 8, ferner umfassend die folgende Komponente (F):
Komponente (F): ein festes alkalisches Mittel.

10. Pulverförmige Zusammensetzung gemäß Anspruch 9, wobei die Komponente (F) Natriummetasilicat ist.

11. Haarfärbe- oder Haarbleichset, zusammengesetzt aus einer ersten Zusammensetzung, bestehend aus der pulverförmigen Zusammensetzung, wie in einem der Ansprüche 1 bis 10 definiert, und einer zweiten Zusammensetzung, die Wasserstoffperoxid umfasst.

12. Verwendung einer Mischung von der pulverförmigen Zusammensetzung gemäß einem der Ansprüche 1 bis 10 und einer Zusammensetzung, umfassend Wasserstoffperoxid, zum Färben oder Bleichen von Haaren.

13. Verfahren zur Herstellung der pulverförmigen Zusammensetzung gemäß einem der Ansprüche 1 bis 10, umfassend das Mischen der Komponenten (A) bis (C), sodass die Komponenten (A) und (B) auf der Komponente (C) geträgert werden, und das Mischen der Komponente (D) damit.

14. Verfahren zum Färben oder Bleichen von Haaren, umfassend die folgenden Schritte (a) bis (c):

(a) das Mischen einer ersten Zusammensetzung, bestehend aus der pulverförmigen Zusammensetzung, wie in einem der Ansprüche 1 bis 10 definiert, und einer zweiten Zusammensetzung, umfassend Wasserstoffperoxid, um eine Mischung herzustellen;
(b) das Auftragen der Mischung auf die Haare und das Belassen der Haare für 1 bis 45 Minuten;
(c) das Waschen der Haare zum Ausspülen der Mischung.

## Revendications

**1.** Composition pulvérulente comprenant les composants (A), (B), (C) et (D) suivants, dans laquelle un rapport molaire du composant (B) au composant (A), (B)/(A), est de 0,1 ou plus et 10 ou moins et la teneur en eau est de 10 % en masse ou moins :

Composant (A) : une alcanolamine présentant une valeur SP, selon le procédé Fedors, de 10 $(cal/cm^3)^{1/2}$ ou plus et 16 $(cal/cm^3)^{1/2}$ ou moins ou un sel de celui-ci ;
Composant (B) : un composé présentant une valeur SP, selon le procédé Fedors, de $\pm 6$ $(cal/cm^3)^{1/2}$ incluant la valeur SP du composant (A) en tant qu'alcanolamine, à condition que ces composés correspondant au composant (A) soient exclus du composant (B) ;
Composant (C) : un support poudreux ;
Composant (D) : un halogénure d'ammonium.

**2.** Composition pulvérulente selon la revendication 1, dans laquelle le mélange du composant (A) et du composant (B) est supporté sur le composant (C).

**3.** Composition pulvérulente selon la revendication 1, dans laquelle le composant (D) est enrobé d'un composé polymère hydrophile.

**4.** Composition pulvérulente selon l'une quelconque des revendications 1 à 3, dans laquelle le composant (B) est un diol aliphatique ou un diol aromatique.

**5.** Composition pulvérulente selon l'une quelconque des revendications 1 à 4, qui est utilisée pour décolorer les cheveux sous forme de mélange avec une composition contenant du peroxyde d'hydrogène.

**6.** Composition pulvérulente selon l'une quelconque des revendications 1 à 4, comprenant en outre le composant (E) suivant :
Composant (E) : un colorant direct.

**7.** Composition pulvérulente selon la revendication 6, dans laquelle le composant (E) est un ou plusieurs colorants azoïques sélectionnés dans le groupe consistant en (E-1), (E-2) et (E-3) ci-dessous.

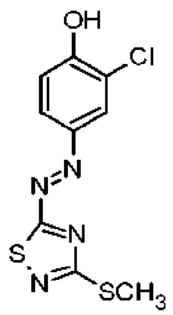
(E-1)

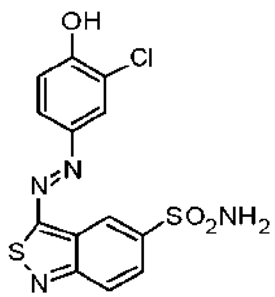
(E-2)

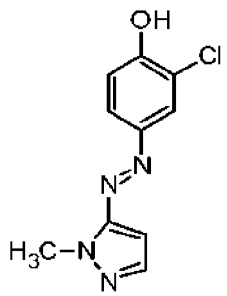
(E-3)

**8.** Composition pulvérulente selon la revendication 6 ou la revendication 7, qui est utilisée pour teindre les cheveux sous forme de mélange avec une composition contenant du peroxyde d'hydrogène.

**9.** Composition pulvérulente selon l'une quelconque des revendications 1 à 8, comprenant en outre le composant (F) suivant :
Composant (F) : un agent alcalin solide.

**10.** Composition pulvérulente selon la revendication 9, dans laquelle le composant (F) est du métasilicate de sodium.

**11.** Kit de teinture ou de décoloration capillaire composé d'une première composition consistant en la composition pulvérulente telle que définie dans l'une quelconque des revendications 1 à 10 et d'une seconde composition comprenant du peroxyde d'hydrogène.

**12.** Utilisation d'un mélange de la composition pulvérulente selon l'une quelconque des revendications 1 à 10 et d'une composition comprenant du peroxyde d'hydrogène pour teindre ou décolorer les cheveux.

**13.** Procédé de fabrication de la composition pulvérulente selon l'une quelconque des revendications 1 à 10, comprenant le mélange des composants (A) à (C) de sorte que les composants (A) et (B) sont supportés sur le composant (C), et le mélange du composant (D) avec ceux-ci.

**14.** Procédé de teinture ou de décoloration des cheveux comprenant les étapes (a) à (c) suivantes :

(a) le mélange d'une première composition consistant en la composition pulvérulente telle que définie dans l'une quelconque des revendications 1 à 10 et d'une seconde composition comprenant du peroxyde d'hydrogène pour préparer un mélange ;
(b) l'application du mélange sur les cheveux et le fait de laisser les cheveux reposer pendant 1 à 45 minutes ;
(c) le lavage des cheveux pour rincer le mélange.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- FR 3015232 A1
- JP 2007320923 A
- JP 2016011297 A